# EUROPEAN PATENT SPECIFICATION

(11) **EP 1 257 304 B1**
(45) Date of publication and mention of the grant of the patent: **04.02.2004**
(21) Application number: 01912909.7
(22) Date of filing: 21.02.2001
(51) Int. Cl.: A61L 15/42, A61L 15/12, A61L 26/00

(54) **FOAM-FORMING WOUND DRESSING**
SCHAUMBILDENDER WUNDVERBAND
PANSEMENT A FORMATION DE MOUSSE

(30) Priority: 25.02.2000 US 184997 P
(43) Date of publication of application: 20.11.2002
(73) Proprietor: Sierra, David, Aptos, California 95003 (US)
(72) Inventor: Sierra, David, Aptos, California 95003 (US)
(74) Representative: Sexton, Jane Helen
(86) International application number: PCT/US2001/005624
(87) International publication number: WO 2001/062312

(56) References cited:
- EP-A- 0 747 420
- WO-A-01/05443
- WO-A-96/17595
- WO-A-98/02098
- WO-A-99/66964
- US-A- 4 427 651
- US-A- 5 583 114
- US-A- 5 733 563

## Description

### FIELD OF THE INVENTION

This invention relates to foam-forming wound dressing materials and methods to prepare and use such materials.

### BACKGROUND OF THE INVENTION

Large scale burn wounds and other open wounds with skin loss often require treatment with temporary skin dressing. The purpose of temporary dressings is to provide immediate coverage and protect the wound from further injury; reduce the rate of evaporative water loss and associated heat loss; prevent or minimize infection; absorb materials exuded from the wound, including dead leukocytes, epidermal and dermal cells; and encourage vascularization and tissue regeneration in the wound. These factors are particularly relevant to traumatic wounds and burns such as those that occur in the field away from emergency medical treatment.

Traditionally, wounds have been encouraged to heal by placing a gauze material over the wound. More recently, a number of products have been introduced in the market that have been used as temporary wound dressings. These include, for example, gels, synthetic foams, and acrylic polymer spray solutions. However such dressings are expensive to produce and/or fail to provide sufficient advantages over traditional dressings. Thus, a need exists for temporary a wound dressing that is low cost, easy to use, and provides superior properties over conventional dressings.

### SUMMARY OF THE INVENTION

Accordingly, the present invention provides a foam wound dressing, comprising albumin and at least one additional protein.

The present invention also provides a composition for preparing a wound dressing that expands into a foam when subject to a shearing force, said composition comprising albumin and at least one additional protein that assists to expand the foam when a shear force is applied to the composition.

Additionally, the invention provides many different embodiments of these compositions, including, for example, inclusion of human proteins, lysozyme, a metal chelating protein, and/or a biologically active agent to the composition.

The present invention further provides a method of preparing a foam wound dressing, said method comprising the steps of:
a) preparing a liquid solution of proteins comprising albumin and at least one additional protein; and
b) subjecting the solution to a shearing force to generate the foam.

### DETAILED DESCRIPTION OF THE INVENTION

In view of the deficiencies of the prior art wound dressing compositions, it is an objective of the invention to provide a sterile and inexpensive wound dressing composition which is a liquid before use to facilitate application to the wound, but which, after being applied, forms a stable foam in situ to protect the wound and maintain a moist environment at the tissue surface. Another object is to provide a dressing that is shelf stable, yet easily prepared and requires no refrigeration. The present invention provides new and useful compositions for achieving these desirable objectives.

### Wound Dressing Composition

The present invention is based on the finding that particular proteins can form a relatively stable space-filling foam when a solution containing the proteins is subject to a shearing force. Such wound dressing compositions comprise proteins which may be obtained from natural sources, from cells transformed with recombinant DNA encoding the protein, or produced synthetically.

Albumin is generally present in the composition at a concentration of about 6 g/L and up to a 40 g/L. However, the actual concentration of albumin can vary somewhat depending on the other proteins or additives included in the composition. The optimal concentration of albumin can readily be determined without undue experimentation by varying the albumin concentration and then measuring the volume expansion index of the foam essentially as described in Example 1.

Albumin useful for the wound dressing compositions of the present invention can be obtained from serum or plasma of an animal, including, for example, from a human, cow, horse, sheep, goat or pig. Human albumin is a preferred albumin to be used in wound dressings intended for human use because this protein is non-immunogenic in humans. Pooled outdated plasma from human blood donors is a particularly economic source of starting material from which to obtain human albumin. Ovalbumin from chicken egg whites also can be used in place of an animal albumin source.

Methods to isolate albumin in relatively pure form from plasma or serum are well known in the art. Large scale commercial methods are available including the alcohol precipitation method of Cohn, described in U.S. Pat. Nos. 2,390,074 and 2,469,193 (see also J. Amer. Chem. Soc. 68, 469-75 (1946); Kirk-Othmer, Encyl. of Chem Tech., 3, 584-88 (2d. ed.1964)) and the plasma protein fraction of Hink (U.S. Pat. No. 2,958,628). Albumin can be purified from Cohn Fraction IV-1 and Cohn Fraction IV-4, or Cohn fraction V precipitate.

Other useful methods to obtain albumin in relatively purified form include, for example, PEG fractionation (e.g., U.S. Pat. No. 3,415,804), fractionation with pluronics (e.g., U.S. Pat. No. 3,850,903), salt fractionation such as by saturated ammonium sulfate, ion exchange chromatography (e.g., U.S. Pat No. 4,228,154; using Cohn fraction II+IIIS cryosupernatant), hydrophobic chromatography, size exclusion chromatography, and electrophoretic separation or by various combinations of the above methods.

Albumin also can produced using recombinant DNA technology and purified from recombinant cells or from the media of recombinant cells, as described, for example, in U.S. patent Nos. 5,986,062 (Ohmura) 5,962,649 (Noda et al.,), 5,849,874 (Van der Laken et al.) or 5,759,819 (Kobayashi et al.).

The wound dressing compositions of the invention include, in addition to albumin, at least one other protein that helps to expand the volume of foam. Although one does not need to know the mechanism by which proteins of the composition form a relatively stabilized wound dressing foam, without being bound by any theory, it is believed that stabilization takes place by crosslinking or polymerizing the proteins, which result in strengthening walls that surround and trap gas bubbles in the foam. By addition of at least one protein other than albumin, the foam should expand from 4 to 8 times the volume of the liquid composition (i.e. a 4 to 8 volume expansion index).

Crosslinking or polymerization of proteins in the wound dressing composition is achieved by chemical or non-chemical means. If chemical crosslinking is contemplated, any of a variety of chemicals crosslinkers can be used, including, for example, homobifunctional or heterobifunctional crosslinking reagents, many of which are commercially available (see, e.g., Pierce Chemical Co. or Sigma Chemical Co.). Crosslinking can be achieved by any of a variety of chemistries well known in the art including, for example, activated polyethylene glycols, aldehydes, isocyanates, maleimides and the like.

Chemical crosslinking allows the inclusion of any of a wide variety of proteins with albumin, provided such proteins can react chemically with the crosslinker. Such proteins include, for example, serum proteins broadly classified as alpha, beta or gamma globulins, as well as gelatin and collagen.

Expansion and stabilization of the foam by crosslinking or polymerization .also can be achieved by non-chemical means. For example, application of a shear force to the liquid composition denatures proteins, which then polymerize, supporting expansion of the foam. Proteins suitable in this regard include metal binding proteins such as transferrin, ferriten, conalbumin, and other proteins that can be crosslinked or polymerize upon denaturation, including, for example, lysozyme and ovomucin.

The present invention provides a composition for preparing a wound dressing that expands into a foam when subject to a shearing force, said composition comprising albumin and at least one additional protein selected from lysozyme, a metal binding protein and ovamucin.

Transferrin can be obtained from animal plasma or animal serum essentially using methods well known in the art including, for example, U.S. patent no. 5,744,586 (Rolf et al.), 5,041,537 (Bethke et al.). Cohn Fraction IV, often considered a waste product of the Cohn fractionation process, contains a range of useful plasma proteins including albumin, alpha-1 proteinase inhibitor (also known as alpha-1 antitrypsin) and transferrin. Therefore, Fraction IV of the Cohn fractionation process (specifically fractions IV-1 and IV-4) can serve as a starting source for purification of transferrin (see, e.g., J. K. Inman et al (Vox Sang. 6:34 (1961); transferrin approximately 93% pure obtained from Cohn's alcohol precipitate fraction IV paste).

Lysozyme can be obtained from mammalian sources, including tissues and body fluids such as blood, leukocytes, tears, and milk, from avian sources, such as avian egg-white, and from vegetable sources. Lysozyme can be purified by a variety of methods well known in the art including, for example, methods described in U.S. Patent Nos. 3,940,317 (Katz et al.), 4,504,583 (Hasakawa et al.,; purification from egg white by crystallization) 4,552,845 (Reid et al.; purification from egg white by ion exchange), 4,945,051 (Kikuchi et al., human recombinant lysozyme) and 5,618,712 (Sledziewski - human recombinant lysozyme. Lysozyme also can be obtained from mammalian serum or plasma that have been first processed by the Cohn fractionation method (e.g., Cohn's fraction IV-1) or by ammonium sulfate fractionation (see, e.g., U.S. Patent no. 4,104,125, describing the purification of human lysozyme by ion exchange chromatography). Although not wishing to be bound by any mechanism, it is believed that lysozyme can provide anti-microbial properties through its ability to lyse bacterial cell walls.

Conalbumin, ovomucin, and lysozyme can be obtained from egg whites. Methods to purify these components from egg white are well known in the art. Methods to purify gelatin and collagen also are well known.

An preferred foam forming wound dressing composition comprises albumin, transferrin and lysozyme. In a preferred embodiment, albumin is present at 6 g/L, transferrin at 2.2 g/L, and lysozyme at 0.3 g/L. The composition can include sodium chloride at the concentration of saline (i.e., 0.85%) and a pH of about 8-9. In addition, the tartaric acid can be added to about 0.15 g/L.

Expansion and stabilization of the foam can be achieved by controlling the pH of the composition, by the addition of an acid to a obtain a pH between about 8 to 9, more preferably at about pH 8. Although knowledge of the mechanism by which mildly-basic pH stabilizes the foam is not necessary to achieve stabilization of the wound dressing, without being bound by any theory, it is believed that a mildly basic pH denatures albumin and other proteins which increases their ability to polymerize and stabilize walls surrounding the gas bubbles that are present in the foam. Any acid, including organic and inorganic acids, is useful to reduce the pH of the wound dressing composition. Tartaric acid is a preferred acid and can generally be included in the composition at a concentration between about 0.1 to 0.3 g/L, more preferably about 0.15 g/L.

Expansion and stabilization of the foam also can be achieved by including a metal ion in the composition when the composition includes a metal binding protein, such as a transferrin or conalbumin. Metal ions that can stabilize the foam include, for example, copper and zinc, but not iron. Other metal ions suitable for stabilizing the foam can readily be identified without undue experimentation by measuring the foam volume expansion index as disclosed in Example 1.

Metal ions can be added to the wound dressing composition as a metal salt, such as a metal-chloride salt, generally at a concentration between about 0.1 mM to 1 mM, more preferably about 0.4 mM. The optimal amount of metal ion to use, however, depends on the other components of the composition and can be readily determined by varying the concentration and measuring the resulting foam volume [and strength?] as described in Example 1.

Once the foam is formed, it can be stabilized further by the addition of heat in the order of about 65°C to 70 °C. Heating can be achieved by an external heat source such as by application of a suitable "heat wrap," which is commonly included in emergency medical kits. Although knowledge of the mechanism by which heat stabilizes the foam is not necessary to achieve stabilization of the wound dressing, without being bound by any theory, it is believed that denatures albumin and other proteins which reinforces walls surrounding the trapped gas in the foam.

If heat is to be used, the wound dressing composition also can include a sugar, which helps prevent drainage or collapse of the heated foam by delaying gas evaporation from the bubbles until the heat has accomplished its foam stabilizing effect. A variety of sugars can be used for this purpose including simple and complex sugars well known in the art, such as sucrose, glucose, lactose and the like. In addition, if heat is to be applied the wound dressing also can include agents to assist in uniform spread of the thermal energy throughout the dressing, such agents including for example, agents analogous to porphyrins and the like used in laser solders (see, e.g., Oz et al., J. Vasc. Surg., 11:718-25 (1980)).

The proteins and any other components of the composition for forming a wound dressing are dissolved in a liquid medium before formation of the wound dressing. Such liquid medium is generally aqueous-based, but also can include non-aqueous solvents provided such solvents do not inhibit foam generation or stability or irritate the wound when applied. The composition also can include a compatible buffer to maintain the pH of the solution, generally between about pH 8 to 9. Such buffers are well known in the art and include, for example, Tris, phosphate and carbonate buffers. The wound dressing composition also can include a salt such as a sodium chloride at the concentration of saline (i.e., 0.85%).

The wound dressing composition also can include any of a variety of biologically active agents to protect the wound from infection, further injury or to initiate therapy. Infection preventing biologically active agents include, for example, antiviral, antibacterial, antiparasitic, and antifungal substances and combinations thereof. Specific examples include, but are not limited to: Antibacterial agents such as penicillins, cephalosporins, vancomycin, bacitracin, cephalosporins, polymxyins, amikacin, doxycycline, nystatin, amphotericin-B, tetracyclines, chloramphenicol, erythromycin, neomycin, streptomycin, kanamycin, gentamicin, tobramycin, clindamycin, rifampin, nalidixic acid, flucytosine, griseofulvin, and the like; Antiviral agents such as vidarabine, acyclovir, ribavirin, amantadine hydrochloride, interferons, dideoxyuridine, and the like; Antifungal agents such as nystatin, gentamicin, miconazole, tolnaftate, undecyclic acid and its salts, and the like; and Antiparasitic agents such as quinacrine, chloroquine, quinine, and the like. Antimicrobial properties of the wound dressing composition can be assessed in animal models essentially as described by Longo et al. *J. Pharma. Sci.,* 63;1376, (1974)) or as described in Example 1.

Examples of other biologically active agents include anaesthetic agents such as cocaine, benzocaine, novocaine, lidocaine, bupivocaine, and the like; Analgesic agents such as salicylic acid, salicylate esters and salts, acetaminophen, ibuprofen, morphine, phenylbutazone, indomethacin, sulindac, tolmetin, zomepirac, and the like; Hormonal compounds such as insulin, FSN, ACTH, testosterone, anti-fertility compounds, estrogen, calcitonin and the like; Enzyme-containing compositions; Cytokines or growth factors such as platelet derived growth factor, insulin-dependent growth factor, transforming factored placentile growth factor and the like; Wound healing or clot inducing agents; Vitamins and vitamin derivatives such as Vitamin A, retinol, retinoic acid, .alpha.-tocopherol (Vitamin E), 7-dehydrochloresterol (Vitamin D), Vitamin K, thiamine riboflavin, niacin, pyridoxine, biotin, antothenic acid, ascorbic acid, choline, inositol, and the like; and extracellular matrix components such as collagen materials, elastin and the like.

### Preparation and Storage of the Composition

The wound dressing composition of the present invention can be prepared in dry form using lyophilized proteins and other dry chemical ingredients and then later reconstituted by addition of an appropriate liquid solution when needed. Alternatively, the wound dressing composition can be prepared as a liquid solution and then stored in that state for later use. Storage is generally suitable at room temperature, which is approximately 23-25°C. However, the liquid composition can be stored frozen or at reduced temperatures, generally at about 4°C to increase the shelf life, if desirable.

The wound dressing composition is preferably prepared with sterile ingredients and under sterile conditions or is sterilized after preparation to avoid contacting the wound surface with potentially infectious agents. Methods to sterilize protein containing compositions are well known in the art and include, for example, sterilization by filtration or by addition of microbiocidal agents.

Agents that have a relatively short half-life in liquid solution such as a biologically active agent or chemical cross-linking agents may be stored separately and then added to the foam dressing composition prior to foam formation.

### Preparation of the Foam

When the liquid wound dressing composition is subject to a shear force, it expands to generate a foam. When properly prepared, the foam expands from about 4 to about 8 times the volume of the liquid composition applied to the wound site (i.e., a volume expansion index of 4 to 8). A suitable shear force can be applied by spraying the liquid under pressure through a spray nozzle, by vigorously shaking the solution in a closed container, by blending the solution with a mixing device or by any other means known in the art. Once applied to the body the foam wound dressing is typically stable for at least one to two hours, and generally degrades fully by about 72 hours. However, the amount of other proteins in the composition such as lysozyme or transferrin relative to the amount of albumin can affect the time that the foam is stable. More stability generally results from increasing the ratio of lysozyme or transferrin to alubmin. Once prepared, the foam maintains at least about 50% of the initial foam volume, more preferably about 70% of its initial foam volume for several hours.

The composition for forming a foam wound dressing is conveniently applied to the wound site by spraying. A spray apparatus useful for applying the composition includes a vessel with a dispensing means for spraying the liquid composition contained within the vessel. The dispensing means can be a pump, a fluid pressurizing component, or an aerosol propellant with associated valve control. Generally, any chemical, mechanical or electronic mechanism for propelling the liquid composition as an atomized spray from the vessel is appropriate as the dispensing means. In one embodiment, a spray apparatus includes the liquid composition of proteins in a vessel with a dispensing means for spray delivery of the liquid composition. In another embodiment, the spray apparatus contains the liquid composition of proteins in a liquid aerosol propellant.

The vessel used for dispensing a spray depends on the method of dispensing the liquid composition. For example, when the dispensing means is an aerosol propellant with a valve and nozzle mechanism, an appropriate vessel must be able to withstand pressure generated therein by the aerosol. Such pressure vessels are usually cylindrical in shape and composed of iron, aluminum alloys, glass or plastic. Other suitable vessels include bottles, tubes, pads and the like.

The dispensing means can be any chemical, mechanical or electrical component which acts to propel the liquid composition as an atomized spray from the vessel. The dispensing means can be a pump, a fluid pressurizing component, a collapsible vessel with a tube or jet, or an aerosol propellant with associated valve mechanisms. A preferred dispensing means is a compatible aerosol propellant with valve mechanism which enables spray delivery of the liquid composition.

The dispensing means of the spray apparatus can be comprised of at least one aerosol propellant. The aerosol propellant can be a liquefied or compressed gas which acts to dispense the liquid composition from a pressurized vessel upon actuation of a valve in the dispensing means. When a liquid aerosol propellant is used, it is preferred that it be substantially miscible with any organic solvent of liquid composition and that the proteins and other ingredients of the composition are compatible with the propellant. However, if the liquid propellant is similar in density to the liquid composition, mild shaking prior to use can result in a uniform atomized spray. In the case of a gaseous aerosol propellant, it is preferred that the gaseous propellant be at least partially soluble in any organic solvent of liquid composition.

Suitable liquefied aerosol propellants include blends of hydrocarbons, fluorochlorohydrocarbons, and chlorinated hydrocarbons. Examples of fluorochlorohydrocarbons include trichloromonofluoromethane, dichlorodifluoromethane, dichloromonofluoromethane, 2-tetrafluoroethane, 1,1-dichloro-1,2,2-tetrafluoroethane, 1-chloro-1,1-difluoroethane, 1,1-difluoroethane, and octofluorocyclobutane, and mixtures thereof. Examples of hydrocarbons include liquefied petroleum gases such propane, isobutane, and N-butane and mixtures thereof. The compressed gas propellants are preferably non-toxic, non-flammable, and inert. Examples include carbon dioxide, nitrous oxide and N₂ and the like.

The aerosol propellant is present within the vessel so that it is either mixed or, in the case of a gaseous propellant, in contact with the liquid composition. Alternatively, the liquid composition can be held within a chamber separated from the propellant by a barrier which responds to pressure exerted by the propellant to deliver the liquid composition. In another alternative embodiment, the spray apparatus can have a co-dispensing valve and system so that two ingredients can be kept separate until dispensed. The product components are dispensed in stoichiometric amounts and thoroughly mixed as they are delivered to the tissue site. For example, the liquid composition of foam dressing in a solvent can be held separate from a biologically active agent until they are co-dispensed and mixed upon delivery.

The present invention provides use of a liquid solution of proteins comprising albumin and at least one additional protein in the manufacture of a medicament for dressing a wound wherein the dressing is a foam sound dressing prepared in situ on the surface of the wound.

In addition to spraying the wound with the composition to form the foam wound dressing in situ, an alternative is to "pre-form" the foam dressings which can be packaged and used when needed. In this case, a shear force is applied to the liquid composition to generate the foam, for example, by shaking the liquid in a closed container or by beating or blending the liquid using a rotating mixing device. The pre-formed foam can be further stabilized by applying heat as described for forming the dressing in situ.

Thus the present invention provides use of a liquid solution of proteins comprising albumin and at least one additional protein in the manufacture of a medicament for dressing a wound wherein the dressing is a foam wound dressing which is pre-formed and then applied to the wound.

### Uses of the wound dressing

The wound dressings of the invention have many applications in the fields of human and veterinary medicine. These form-in-place dressings are particularly useful for the initial treatment of wounds involving high to moderate tissue loss, such as occur in battlefield or other dangerous occupations. In such cases, the wound dressing device can be used to cover and close an open wound with moderate bleeding once large arterial gushing is attenuated by tourniquet or clamping. Optionally, the wound dressing may include proteins of the coagulation cascade such as thrombin and fibrinogen to stop the flow of blood and retain the wound dressing.

Foam wound dressings of the invention also may be used for problem wounds such as burns which require an immediate covering to avoid contamination and to reduce further tissue injury. Also, the dressings may be used to fill a cavity such as the peritoneum to provide support and hemostasis to organs such as the liver and spleen, damaged in blunt trauma. Hemostasis is effected by pressure exerted by the expanded foam pressing against the lesion. Hemostatic agents of the foam such as thrombin may be added to the formulation to augment the hemostatic effect of the foam.

Depending on the active agents included in the wound dressing and the time that it is left in contact with the wound, the foam dressing can be used to stimulate desirable cellular responses such as fibroplasia, angiogenesis and epithelialization. Furthermore, these dressings can also be used as a vehicle for the sustained release of medicaments which enhance healing, such as growth factors and antimicrobials, to the wound site simply by inclusion of these active agent medicaments into the composition prior to foam formation.

The foamed wound dressing of the invention can be made entirely biocompatible by using proteins that are derived from the same species of animal which the foam dressing is to be applied. Thus, a foam dressing composition that is biocompatible in humans is produced with human albumin and other human proteins.

### EXAMPLES

### Example 1

### Physical Methods to Analyze the Foam Dressing

### Physical properties of the foam:

During the course of the development of the foam a simple qualitative test is used to compare the adherence properties of different foam formulations: Thin layers of foam are sprayed from aerosol containers held 10 cm from 10 x 10 cm sheets of 0.5 mm thick cast collagen or harvested procine dermal graft. These are then dried at 40°C, cooled and stapled into cylinders. Foams that remain attached to the polythene without peeling or cracking are regarded as sufficiently adherent and elastic for application to body surfaces.

### In vitro drainage and vapor transmission properties of foam:

Foams are sprayed onto U.S. standard testing sieves (No. 60 250 µm opening) leveled off at a depth of 3 cm and covered. Rates of drainage are followed at room temperature by collecting and weighing the drained solution at regular intervals. After 4 hr drainage, the foams are uncovered and placed in an oven for drying to constant weight at 40°C. The dried foams are then used for determinations of their vapor transmission properties. A distilled water reservoir is weighted and sealed onto the grid. The apparatus is placed in a drying oven and transmission of water vapor through the foam layer determined gravimetrically at 33° C. Rates of vapor transmission through metalline are also measured for comparative purposes.

### Foam Volume Expansion Index:

Foam volume expansion index can be used to evaluate the performance of a particular foam composition. In addition, it is useful to determine if a particular protein can expand the foam and to determine an optimal concentration of the protein.

Foam volume expansion index is defined as the volume of the foam over the volume of the liquid used to generate the foam. Generally a foam should expand from 4 to 8 times larger than the volume of the liquid composition used to form the foam, resulting in an expansion index of 4 to 8.

Foam volume can be measured directly by visual inspection of the volume when the foam is formed in a transparent graduated container. A more accurate estimate of foam volume can be obtained indirectly. In this case, the foam is formed in a transparent graduated container and a liquid that does not disturb the foam (e.g., water) is added to the container to achieve a measurable level of volume or "total volume." The added liquid is decanted, its volume determined and then subtracted from the total volume, thus generating a volume of the foam.

### Effect of foam on water evaporation from wounds in vivo:

An area of 4x4 cm of full depth skin is excised from the shaved backs of anaesthetized rats. Square plexiglas chambers (4x5x5 cm) with inlet and outlet tubes are sealed over the excised areas. Air is passed through a molecular sieve (B.D.H. type 4A aluminum sodium silicate) and then passed through the containers at 270 cm³ min⁻¹. Uniform rates of air flow are obtained by using adjustable flow meters attached to the inlet and outlet tubes of the perspex chambers. Air leaving the containers is passed through U-tubes containing sufficient molecular sieve to adsorb all the water vapor released during the experiment. Water vapor trapped by the molecular sieve is determined by weighing at regular intervals. Two rats with excision wounds are used in parallel during each experiment: one rat is used as a control and the wound on the other rat is covered with a 1 cm deep layer of foam. Measurements of vapor losses from the excision wounds are begun 1 hr after foam application by which time the foam layers were almost dry.

### Antimicrobial properties of foam matrix and solution:

Assay of bacteriostatic effects of the foam can be accomplished by zone inhibition. Center wells 2.7 cm in diameter are removed from nutrient agar plates and filled with previously prepared foam samples. Hospital isolates of *Pseudomonas aeruginosa, Staphylococcus aureus,* and *Candida albicans* are inoculated onto appropriate nutrient agar plates and incubated for 24 hr at 37°C. Results are the means of six determinations of the total width of bacteria-free zones excluding the center wells. Population densities of the microbial inocula used in the experiment are determined by serial dilution and plating onto suitable agar medium. Colonies are counted using a dark field colony counter (New Brunswick Scientific Co., Model C 100).

To assay the bactericidal efficiency of foam solutions serial dilutions of the foam are incubated with bacterial inocula of 10⁴ organisms/ml for 24 hr and then plated for assay of colony development. Alternatively, full and half-strength dilutions of foam solution are inoculated with bacterial cultures. Samples taken after 0.5, 15, 30, 60, and 120 min are diluted into nutrient solution to remove bacteriostatic effects. In control treatments microbes are inoculated directly into nutrient solution. Diluted cultures are plated onto suitable agars and any colony development assayed after 24 hr at 37°C.

Antibacterial effects of foam on burn wounds can be tested in vivo on rats. Four tertiary bum wounds are made on the shaved backs of anesthetized rats by application of flame heated 1 cm diameter glass disks for 10 sec. The wounds are covered with a large piece of gauze to facilitate subsequent removal of treatments. Treatments consist of separate inoculation of each wound surface with 1 ml of *Staphylococcus aureus* (10⁸ organisms ml⁻¹) applied onto the absorbent gauze pad. Sterile saline is applied to control treatments. Treatments vary through application of foam either before or after contamination with bacterial cultures. Results are the means of at least four separate determinations.

The level of contamination in inoculated and control wounds is determined by first removing the gauze sheets plus treatments and then excising 3 cm² sections of wounded ski to a depth of 0.5 cm. The excised sections are aseptically transferred to 150 ml of cold nutrient solution in order to dilute out residual antimicrobial activity of foam. The flasks are then incubated overnight at 4°C in order to facilitate release of organisms into the culture solution, while inhibiting substantial levels of cell division. Samples of the solution are then serially diluted, plated, and incubated before assaying microbial populations. Between four and eight replicates are made for each treatment.

The examples set forth above are provided to give those of ordinary skill in the art with a complete disclosure and description of how to make and use the preferred embodiments of the compositions, and are not intended to limit the scope of what the inventors regard as their invention. Modifications of the above-described modes for carrying out the invention that are obvious to persons of skill in the art are intended to be within the scope of the following claims. All publications, patents, and patent applications cited in this specification are incorporated herein by reference as if each such publication, patent or patent application were specifically and individually indicated to be incorporated herein by reference.

## Claims

1. A foam wound dressing, comprising albumin and at least one additional protein.

2. The wound dressing of claim 1, wherein said albumin is human albumin.

3. The wound dressing of claim 1, wherein said at least one additional protein is lysozyme.

4. The wound dressing of claim 1, wherein said at least one additional protein is a metal chelating protein.

5. The wound dressing of claim 4, wherein said metal chelating protein is transferrin.

6. The wound dressing of claim 4, further comprising calcium ions.

7. The wound dressing of claim 1, further comprising an acid.

8. The wound dressing of claim 7, wherein said acid is tartaric acid.

9. The wound dressing of claim 1, further comprising a buffer.

10. The wound dressing of claim 1, further comprising a biologically active agent.

11. A composition for preparing a wound dressing that expands into a foam when subject to a shearing force, said composition comprising albumin and at least one additional protein selected from lysozyme, a metal binding protein and ovamucin.

12. The composition of claim 11, wherein said at least one additional protein is lysozyme.

13. The composition of claim 11, wherein said at least one additional protein is a metal binding protein.

14. The composition of claim 13, wherein said metal binding protein is transferrin.

15. The composition of claim 11, further comprising tartaric acid.

16. A method of preparing a foam wound dressing, said method comprising the steps of:
a) preparing a liquid solution of proteins comprising albumin and at least one additional protein; and
b) subjecting the solution to a shearing force to generate the foam.

17. The method of claim 16, wherein said shearing force results from forcing the solution through a nozzle of a spraying device.

18. The method of claim 16, wherein said solution of proteins is stored lyophilized and later reconstituted before use.

19. The method of claim 16, wherein said at least one of additional protein is lysozyme.

20. The method of claim 16, wherein said at least one of additional protein is a metal binding protein.

21. The method of claim 20, wherein said metal binding protein is transferrin.

22. The method of claim 20, wherein said solution further comprises a metal ion that is chelated by the metal binding protein.

23. The method of claim 16, wherein said solution further comprises an acid which reduces the pH of the solution.

24. The method of claim 16, wherein said solution further comprises a biologically active agent.

25. The method of claim 16, further comprising the step of applying a source of heat to the foam to further strengthen the foam.

26. Use of a liquid solution of proteins comprising albumin and at least one additional protein in the manufacture of a medicament for dressing a wound wherein the dressing is a foam wound dressing prepared in situ on the surface of the wound.

27. Use according to claim 26, wherein the foam is formed by spraying the liquid solution onto the wound.

28. Use of a liquid solution of proteins comprising albumin and at least one additional protein in the manufacture of a medicament for dressing a wound wherein the dressing is a foam wound dressing which is preformed and then applied to the wound.

## Patentansprüche

1. Schaumwundverband, umfassend Albumin und mindestens ein zusätzliches Protein.

2. Wundverband nach Anspruch 1, wobei das Albumin menschliches Albumin ist.

3. Wundverband nach Anspruch 1, wobei das mindestens eine zusätzliche Protein Lysozym ist.

4. Wundverband nach Anspruch 1, wobei das mindestens eine zusätzliche Protein ein metallchelatbildendes Protein ist.

5. Wundverband nach Anspruch 4, wobei das metallchelatbildende Protein Transferrin ist.

6. Wundverband nach Anspruch 4, weiterhin umfassend Calciumione.

7. Wundverband nach Anspruch 1, weiterhin umfassend eine Säure.

8. Wundverband nach Anspruch 7, wobei die Säure Weinsäure ist.

9. Wundverband nach Anspruch 1, weiterhin umfassend einen Puffer.

10. Wundverband nach Anspruch 1, weiterhin umfassend ein biologisch aktives Agens.

11. Zusammensetzung zur Zubereitung eines Wundverbands, der sich zu einem Schaum ausbreitet, wenn er einer Scherkraft ausgesetzt wird, die Zusammensetzung umfassend Albumin und mindestens ein zusätzliches Protein aus der Gruppe von Lysozym, metallbindendem Protein und Ovomucin.

12. Zusammensetzung nach Anspruch 11, wobei das mindestens eine zusätzliche Protein Lysozym ist.

13. Zusammensetzung nach Anspruch 11, wobei das mindestens eine zusätzliche Protein ein metallbindendes Protein ist.

14. Zusammensetzung nach Anspruch 13, wobei das metallbindende Protein Transferrin ist.

15. Zusammensetzung nach Anspruch 11, weiterhin umfassend Weinsäure.

16. Methode zur Zubereitung eines Schaumwundverbands, wobei die Methode die folgenden Schritte umfaßt:
a) Zubereitung einer flüssigen Proteinlösung umfassend Albumin und mindestens ein zusätzliches Protein; und
b) Anwendung einer Scherkraft auf die Lösung, um den Schaum zu bilden.

17. Methode nach Anspruch 16, wobei die Scherkraft durch das Pressen der Lösung durch eine Düse einer Sprühvorrichtung resultiert.

18. Methode nach Anspruch 16, wobei die Proteinlösung gefriergetrocknet gelagert wird und später vor dem Gebrauch rekonstituiert wird.

19. Methode nach Anspruch 16, wobei das mindestens eine zusätzliche Protein Lysozym ist.

20. Methode nach Anspruch 16, wobei das mindestens eine zusätzliche Protein ein metallbindendes Protein ist.

21. Methode nach Anspruch 20, wobei das metallbindende Protein Transferrin ist.

22. Methode nach Anspruch 20, wobei die Lösung weiterhin ein Metallion umfaßt, das durch das metallbindende Protein in einem Chelat gebunden ist.

23. Methode nach Anspruch 16, wobei die Lösung weiterhin eine Säure umfaßt, die den pH-Wert der Lösung senkt.

24. Methode nach Anspruch 16, wobei die Lösung weiterhin ein biologisch aktives Agens umfasst.

25. Methode nach Anspruch 16, weiterhin umfassend einen Schritt zur Anwendung einer Wärmequelle, um den Schaum weiter zu stärken.

26. Einsatz einer flüssigen Proteinlösung, umfassend Albumin und mindestens ein zusätzliches Protein, bei der Herstellung eines Arzneimittels zum Verbinden einer Wunde, wobei der Verband ein Schaumwundverband ist, der in-situ auf der Oberfläche der Wunde zubereitet wird.

27. Einsatz nach Anspruch 26, wobei der Schaum durch das Aufsprühen einer flüssigen Lösung auf die Wunde gebildet wird.

28. Einsatz einer flüssigen Proteinlösung, umfassend Albumin und mindestens ein zusätzliches Protein, bei der Herstellung eines Arzneimittels zum Verbinden einer Wunde, wobei der Verband ein Schaumwundverband ist, der vorgeformt ist und dann auf die Wunde aufgetragen wird.

## Revendications

1. Pansement en mousse, comprenant de l'albumine et au moins une protéine supplémentaire.

2. Pansement selon la revendication 1, dans lequel ladite albumine est de l'albumine humaine.

3. Pansement selon la revendication 1, dans lequel ladite au moins une protéine supplémentaire est un lysozyme.

4. Pansement selon la revendication 1, dans lequel ladite au moins une protéine supplémentaire est une protéine de chélation de métal.

5. Pansement selon la revendication 4, dans lequel ladite protéine de chélation de métal est la transferrine.

6. Pansement selon la revendication 4, comprenant en outre des ions calcium.

7. Pansement selon la revendication 1, comprenant en outre un acide.

8. Pansement selon la revendication 7, dans lequel ledit acide est l'acide tartrique.

9. Pansement selon la revendication 1, comprenant en outre un tampon.

10. Pansement selon la revendication 1, comprenant en outre un agent biologiquement actif.

11. Composition pour la préparation d'un pansement qui se dilate en une mousse lorsqu'il est soumis à une force de cisaillement, ladite composition comprenant de l'albumine et au moins une protéine supplémentaire choisie parmi un lysozyme, une protéine de liaison de métal et une ovamucine.

12. Composition selon la revendication 11, dans laquelle ladite au moins une protéine supplémentaire est un lysozyme.

13. Composition selon la revendication 11, dans laquelle ladite au moins une protéine supplémentaire est une protéine de liaison de métal.

14. Composition selon la revendication 13, dans laquelle ladite protéine de chélation de métal est la transferrine.

15. Composition selon la revendication 11, comprenant en outre de l'acide tartrique.

16. Procédé de préparation d'un pansement en mousse, ledit procédé comprenant les étapes consistant à :
a) préparer une solution liquide de protéines comprenant de l'albumine et au moins une protéine supplémentaire ; et
b) soumettre la solution à une force de cisaillement pour générer la mousse.

17. Procédé selon la revendication 16, dans lequel la force de cisaillement est due à l'enfoncement de la solution à travers un tuyau d'un appareil de pulvérisation.

18. Procédé selon la revendication 16, dans lequel ladite solution de protéines est stockée lyophilisée et ensuite reconstituée avant utilisation.

19. Procédé selon la revendication 16, dans lequel ladite au moins une des protéines supplémentaires est un lysozyme.

20. Procédé selon la revendication 16, dans lequel ladite au moins une des protéines supplémentaires est une protéine de liaison de métal.

21. Procédé selon la revendication 20, dans lequel ladite protéine de liaison de métal est la transferrine.

22. Procédé selon la revendication 20, dans lequel ladite solution comprend en outre un ion métallique qui est chélaté par la protéine de liaison de métal.

23. Procédé selon la revendication 16, dans lequel ladite solution comprend en outre un acide qui réduit le pH de la solution.

24. Procédé selon la revendication 16, dans lequel ladite solution comprend en outre un agent biologiquement actif.

25. Procédé selon la revendication 16, comprenant en outre l'étape consistant à appliquer une source de chaleur sur la mousse pour davantage renforcer la mousse.

26. Utilisation d'une solution liquide de protéines comprenant de l'albumine et au moins une protéine supplémentaire pour la fabrication d'un médicament pour recouvrir une blessure, dans laquelle le pansement est un pansement en mousse préparé in situ sur la surface de la blessure.

27. Utilisation selon la revendication 26, dans laquelle la mousse est formée par pulvérisation de la solution liquide sur la blessure.

28. Utilisation d'une solution liquide de protéines comprenant de l'albumine et au moins une protéine supplémentaire pour la fabrication d'un médicament pour recouvrir une blessure, dans laquelle le pansement est un pansement en mousse qui est préformé et ensuite appliqué sur la blessure.
